# FASCICULE DE BREVET EUROPEEN

(11) **EP 1 121 117 B1**
(45) Date de publication et mention de la délivrance du brevet: **05.06.2002**
(21) Numéro de dépôt: 99946400.1
(22) Date de dépôt: 13.10.1999
(51) Int. Cl.: A61K 31/282, A61K 33/24, A61K 9/08, A61K 31/555, A61P 35/00

(54) **CONDITIONNEMENT D'UNE PREPARATION D'OXALIPLATINE**
VERPACKUNG EINES OXALIPLATINHALTIGEN ARZNEIMITTELS
OXALIPLATINUM PREPARATION PACKAGING

(30) Priorité: 14.10.1998 CH 206798
(43) Date de publication de la demande: 08.08.2001
(73) Titulaire: DEBIOPHARM S.A., 1000 Lausanne 9 (CH)
(72) Inventeur: MAUVERNAY, Rolland-Yves, CH-1000 Lausanne 9 (CH)
(74) Mandataire: Nargolwalla, Cyra
(86) Numéro de dépôt international: IB9901670
(87) Numéro de publication internationale: WO0021527

(56) Documents cités:
- WO-A-96/04904
- WO-A-98/39009
- WO-A-99/43355

## Description

La présente invention concerne une poche souple étanche à usage médical contenant une préparation pharmaceutique d'oxaliplatine.

Le complexe optiquement actif du platine cis-oxalato(trans-*l*-1,2-diaminocyclohexane)platine (II) est connu sous la dénomination commune internationale (DCI) de "oxaliplatine" pour posséder des propriétés anti tumorales et sa préparation a été décrite dans le brevet US 4,169,846.

L'oxaliplatine, comme d'autres complexes du platine tels que le cisplatine ou le carboplatine, est utilisé en tant qu'agent anti néoplastique cytostatique pour le traitement thérapeutique de divers types de cancers. Parmi ceux-ci, on peut citer entre autres le cancer du colon, celui des ovaires, des voies respiratoires supérieures ou les cancers épidermoïdes, ainsi que les tumeurs à cellules germinales (testicules, médiastin, glande pinéale, etc...). L'utilisation de l'oxaliplatine est particulièrement appropriée pour le traitement des cancers du colon résistant aux pyrimidines, ceux du poumon à petites cellules, des lymphomes non Hodgkiniens, des cancers du sein, des cancers des voies aérodigestives supérieures, des mélanomes malins, des hépatocarcinomes, des cancers urothéliaux, ceux de la prostate, etc.

La demande internationale WO 96/04904 décrit une préparation pharmaceutique d'oxaliplatine en solution aqueuse. Cette préparation présente l'avantage d'obtenir une solution injectable d'oxaliplatine prête à l'emploi, plus simple et plus sure à utiliser et moins coûteuse à fabriquer qu'une préparation à partir d'un lyophilisat. Elle présente une pureté chimique (pas de racémisation) et une activité thérapeutique équivalente ou supérieure à celles obtenues à partir d'un lyophilisat reconstitué.

Cette préparation pharmaceutique a été conservée dans des flacons en verre neutre pour usage pharmaceutique sous atmosphère d'un gaz inerte. Cependant, de tels flaconnages, s'ils sont appropriés pour la conservation sur une longue période de la préparation pharmaceutique, ne le sont pas pour contenir cette préparation lors d'une administration par perfusion.

Lors d'opérations de perfusion de préparations liquides de complexes du platine autres que l'oxaliplatine tels que le cisplatine ou le carboplatine, on utilise des poches souples constituées d'un matériau à base de chlorure de polyvinyle (PVC).

Toutefois, et contrairement à ce qui a été observé pour des préparations liquides de cisplatine et de carboplatine, il s'est avéré que, du fait en particulier d'une sensibilité chimique plus accrue, les préparations pharmaceutiques d'oxaliplatine en solution aqueuse ne peuvent se trouver au contact de matériaux à base de PVC, ni être transportées et/ou conservées au sein de récipients, en particulier des poches souples, à base de ces matériaux.

Le but de la présente invention est de mettre à disposition des préparations liquides pharmaceutiques d'oxaliplatine qui puissent être non seulement conservées au cours d'une longue période de temps sans qu'aucune perte de qualité ne puisse être constatée, mais également qui puissent être utilisées en particulier pour des opérations de perfusion sans qu'aucune opération de transvasement des préparations liquides pharmaceutiques ne soit à effectuer par le personnel soignant

A cet effet, la présente invention concerne une poche souple étanche à usage médical contenant une préparation pharmaceutique d'oxaliplatine se trouvant sous forme liquide telle que définie à la revendication 1. Les différentes variantes d'exécution sont telles que définies aux revendications 2 à 9.

L'invention sera présentée ci-après à l'aide du dessin dans lequel
- la Fig. 1 représente une vue en coupe de l'enveloppe de la poche souple utilisée dans l'invention;
- la Fig. 2 représente une vue en coupe de la poche souple utilisée dans l'invention;
- la Fig. 3 représente un diagramme comparatif de la perméabilité à l'eau en phase liquide entre, d'une part, le PVC et, d'autre part, un matériau approprié pour constituer l'enveloppe de la poche souple utilisée dans l'invention, avant et après une opération de stérilisation;
- la Fig. 4 représente un diagramme comparatif de la perméabilité à l'eau en phase vapeur de différents matériaux appropriés pour constituer l'enveloppe de la poche souple utilisée dans l'invention, avant et après une opération de stérilisation;
- la Fig. 5 représente un diagramme comparatif de la perméabilité à l'oxygène de différents matériaux appropriés pour constituer l'enveloppe de la poche souple utilisée dans l'invention, avant et après une opération de stérilisation;
- la Fig. 6 représente la variation du pH au cours du temps d'une préparation selon l'invention; et
- la Fig 7 représente la variation du pH au cours du temps d'une solution pharmaceutique liquide d'oxaliplatine conservée dans un flacon en verre sous atmosphère d'un gaz inerte.

La préparation pharmaceutique d'oxaliplatine selon la présente invention est conservée, puis utilisée directement, dans une poche souple et flexible réalisée à partir de matériaux plastiques choisis parmi les polyéthylènes (PE), les polypropylènes (PP), les polyacétates d'éthyle et vinyle, les polyamides (PA), les poly-isobutyles (PIB). Le latex peut également être utilisé.

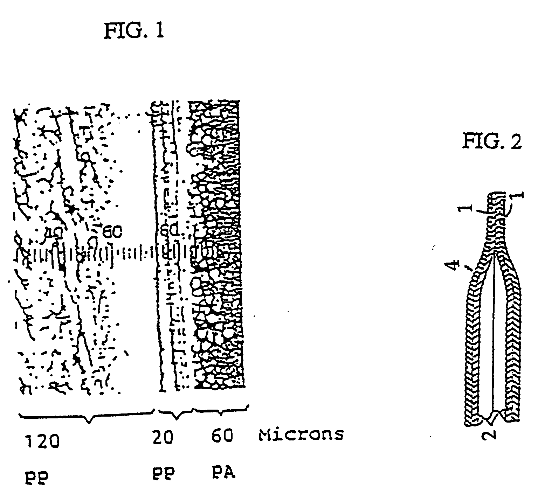

Telle que représentée à la Fig. 1, l'enveloppe de la poche a de préférence une structure multicouche. De préférence encore, la couche interne qui se trouve au contact direct de la préparation pharmaceutique est constituée de PP, la couche externe ou les couches éventuelles intermédiaires pouvant être constituée de l'un quelconque des plastiques mentionnés précédemment. La couche externe ou les couches éventuelles intermédiaires peuvent même être constituées de PVC, l'oxaliplatine ne se trouvant pas au contact direct de ce matériau.

Telle que représentée à la Fig 2, et selon une forme particulière de réalisation de l'invention, la poche souple peut être constituée de feuilles soudées de matériaux multicouches. De préférence, la poche souple peut être constituée par au moins deux feuilles soudées entre elles. On préférera plus particulièrement encore que cette poche puisse être constituée de deux feuilles soudées de matériaux multicouches en feuille comprenant un film de l'acide 11-amino-undécanoïque (PA 11) lié par l'une au moins de ses faces à un film de PP au moyen d'un film de polyoléfine, les films de PP formant la paroi interne de la poche souple étanche.

La poche souple de l'invention est de préférence constituée en un matériau constitué à 70 % de PP et 30 % de PA 11 et communément appelé V90.

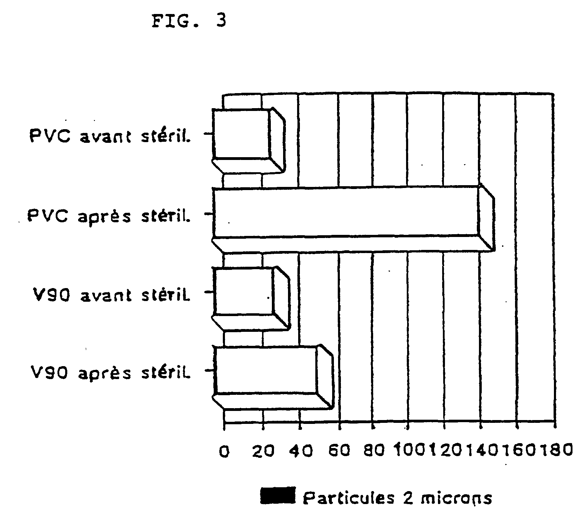

Il a été constaté de manière surprenante, au cours d'une étude physico-chimique menée avant et après opération de stérilisation et comparant les propriétés de perméabilité à l'eau en phase liquide d'enveloppes de poches souples constituées, d'une part, de PVC, d'autre part, de V90, que les poches constituées à base du matériau V90 constituent une excellente barrière à la perte d'eau en général due à l'évaporation. Cette caractéristique ne se retrouve pas dans les poches classiques en PVC, ni même dans celles utilisant le PVC comme constituant de la couche interne. Les résultats de cette étude sont schématisés par le diagramme de la Fig. 3.

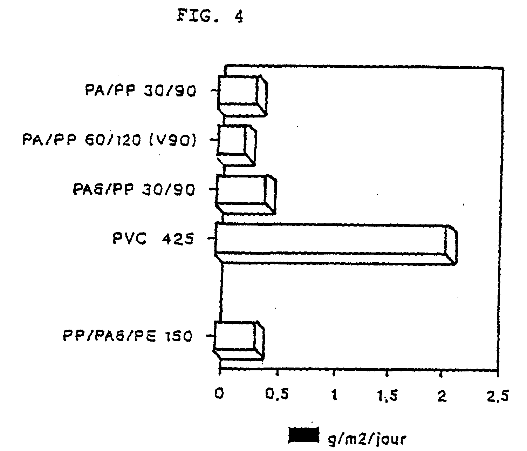

Au cours d'une seconde étude comparant les propriétés de perméabilité à l'eau en phase vapeur du PVC et de différents matériaux constituant les poches souples utilisées pour l'invention, le matériau V90 s'est avéré être le plus étanche comme le montre le diagramme de la Fig. 4.

De telles propriétés d'étanchéité à l'eau sous ses deux formes liquide et gazeuse sont extrêmement importantes pour envisager l'utilisation d'un tel matériau pour la constitution des poches souples utilisées pour l'invention. En effet, les pertes quasiment nulles en eau garantissent la conservation d'une concentration quasiment constante au cours du temps des préparations pharmaceutiques en oxaliplatine.

L'emballage excessif de l'enveloppe de la poche utilisée pour l'invention est alors inutile.

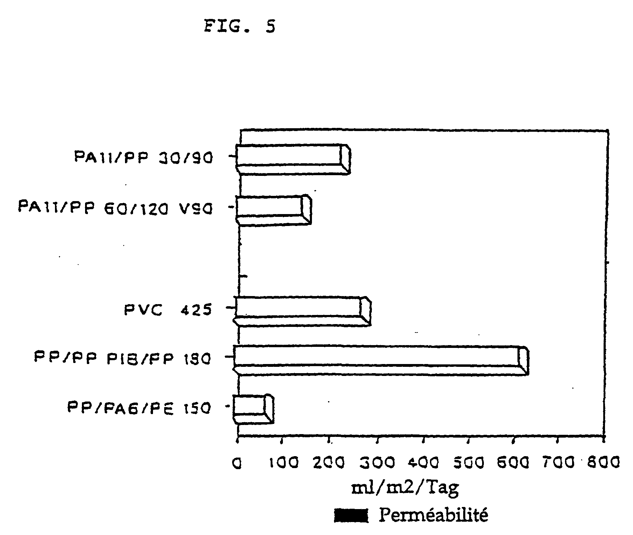

L'étanchéité du matériau V90 vis-à-vis de l'oxygène a également été étudiée et comparée à celle du PVC et elle s'est avérée être bien d'un niveau très supérieur. Les résultats de cette étude comparative sont schématisés sur le diagramme de la Fig. 5.

Cette propriété d'étanchéité à l'oxygène est très importante considérant la sensibilité de l'oxaliplatine aux produits oxydants, les produits de dégradation générés au cours d'une telle oxydation étant en général inactifs du point de vue pharmacologique et peuvent être même toxiques pour l'organisme. Cette qualité est tout à fait appropriée lors de l'utilisation de poche souple qui ont l'avantage, par rapport aux flacons en verre, de ne pas requérir de la présence d'atmosphère de gaz inerte.

Le matériau V90, offre également l'avantage du point de vue écologique d'être recyclé et d'être utilisé sous une autre forme, ce qui n'est pas le cas avec le PVC.

Un autre intérêt d'utiliser les matériaux mentionnés précédemment, et particulièrement le PP (V90), réside dans la possibilité de faire des soudures étanches très simples. Ainsi on peut obtenir des poches planes comportant des compartiments. Cette caractéristique n'est pas réalisable avec un matériau en PVC qui nécessite l'utilisation de connecteurs pour la communication entre les différents compartiments. Ces connecteurs sont malheureusement sources de fuites, ce qui n'est pas constaté dans le cas de poches en PP (V90).

Ces compartiments peuvent être multiples de manière à permettre le mélange de différentes solutions. Ces compartiments peuvent contenir la solution déjà prête à l'emploi, à la bonne dose, et qui peut être prélevée ou utilisée directement par le personnel médical sans risque d'erreur.

Les matériaux mentionnés précédemment, et particulièrement le PP (V90), présentent également l'avantage de mieux supporter les hautes températures. Ceci est particulièrement intéressant lors de la stérilisation de poches souples contenant une solution d'oxaliplatine par autoclave. Cette stérilisation est bien plus facile car en augmentant la température on peut ainsi diminuer le temps d'exposition.

La solution liquide d'oxaliplatine contenue dans ces poches a de préférence une concentration comprise entre 1 et 8 mg/ml. Selon une forme particulière de l'invention, la concentration d'oxaliplatine est comprise entre 1 et 5 mg/ml présentant un pH compris entre 4 et 7, idéalement entre 4,5 et 6. La teneur en oxaliplatine dans la préparation selon une forme particulière de l'invention contient au moins 95 % de la teneur initiale et présente un aspect limpide incolore exempt de précipité après conservation pendant une durée pharmaceutiquement acceptable.

Une étude de la stabilité de la solution liquide d'oxaliplatine (Tanaka K.K. lot LO 92 TO 34) a été réalisée. Pour cela, des poches 100 ml constituées en PA 11/PP 60/140 et de dimension 13,0 x 12,5 cm ont été employées. Les poches contenaient 200 mg d'oxaliplatine à la concentration de 2 mg/ml, soit 100 ml de solution liquide d'oxaliplatine par poche. Cette étude a été réalisée sur un total de 12 semaines suivant le plan d'échantillonnage figurant au dessin 7. Les poches ont été soumises à des conditions de conservation dites accélérées à une température de 40° C et à une humidité relative (HR) de 75 %.

Les résultats de cette étude de stabilité accélérée sont rassemblés dans le tableau 1.

| Cinétique Paramètres | S0 | S1 | S2 | S3 | S4 | S5 | S12 | 6 mois | 12 mois |
|---|---|---|---|---|---|---|---|---|---|
| Aspect de la solution | Incolore limpide | Incolore limpide | Incolore limpide | Incolore limpide | Incolore limpide | Incolore limpide | Incolore limpide | Incolore limpide | Incolore limpide |
| Dosage L-OHP/standard (%) | 99,7 | 100,1 | 100,0 | 100,9 | 99,3 | 97,7 | 99,3 | 98,9 | 98,5 |
| Dosage acide oxalique (%) | < 0,1 % | < 0,1 % | < 0,1 % | < 0,1 % | < 0,1 % | < 0,1 % | < 0,1 % | < 0,1 % | < 0,1 % |
| Impuretés apparentées (%) | 0,10 | 0,60 | 0,50 | 0,50 | 0,48 | 0,45 | 0,40 | 0,5 | 0,6 |
| pH | 5,56 | 5,10 | 5,24 | 5,22 | nM | 5,23 | 5,35 | 5,20 | 5,30 |
| NM = Nom mesuré | | | | | | | | | |

De manière surprenante, la solution liquide d'oxaliplatine conditionnée en poche souple est stable pendant une période allant sur une période bien au-delà de trois mois, et même au-delà de six mois. Etonnamment, cette solution liquide d'oxaliplatine conditionnée en poche apparaît rester stable pendant au moins un an.

L'aspect de la solution a été suivi sur douze mois et a montré à notre grande surprise une limpidité et une absence de coloration sur toute cette période. L'analyse de dosages a été effectuée par chromatographie liquide à haute pression (CLHP ou HPLC).

En ce qui concerne le dosage d'oxaliplatine, on obtient une quantité déclarée comprise entre 95 et 105 % en prenant compte de la limite de résolution du système. Pour ce qui est du dosage d'acide oxalique, la limite maximale est de 0,5 % par méthode HPLC.

De la même manière on détermine un pourcentage d'impuretés apparentées maximal de 2 %.

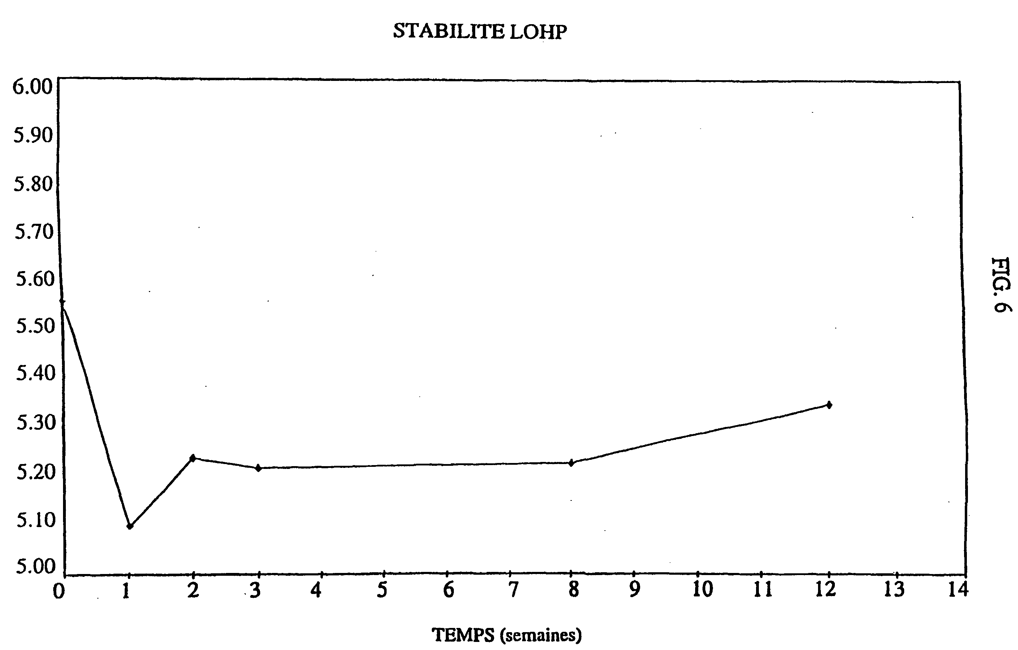

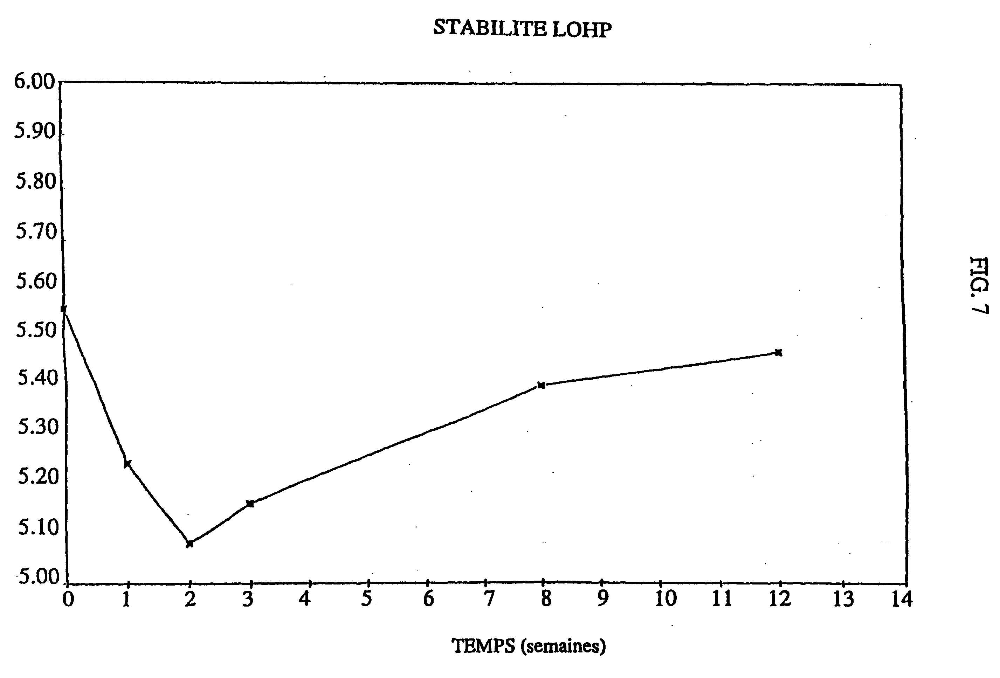

La Fig. 6 montre l'évolution du pH sur 12 semaines. Ce pH est compris entre 4,7 et 5,9 et varie très peu avec le temps, ce qui prouve bien que la solution d'oxaliplatine reste stable dans ce type de poche. Ce système présente une stabilité analogue à celle observée pour une solution d'oxaliplatine soumise aux mêmes conditions et conditionnée en flacon de verre comme le montre la Fig. 7.

Tous les résultats précédents convergent pour montrer que des préparations pharmaceutiques d'oxaliplatine peuvent être conservées, sans qu'aucune dégradation chimique de l'oxaliplatine ne soit observée, sur une longue période dans des poches souples, à partir du moment où elle ne se trouve pas au contact direct d'un matériau à base de PVC. De telles préparations, du fait de la souplesse des matériaux constituant les poches, se trouvent être prêtes à être utilisées pour des opérations de transfusion sans qu'aucune opération de transvasement soit nécessaire.

## Revendications

1. Poche souple étanche à usage médical contenant une préparation pharmaceutique d'oxaliplatine se trouvant sous forme liquide, **caractérisée en ce que** ladite poche souple est réalisée en matière plastique, à l'exclusion, pour ladite matière se trouvant au contact direct de ladite préparation phanataceutique, d'un matériau à base de PVC.

2. Poche souple étanche à usage médical contenant une préparation pharmaceutique d'oxaliplatine selon la revendication 1, **caractérisée en ce que** l'enveloppe de ladite poche a une structure multicouche.

3. Poche souple étanche à usage médical contenant une préparation pharmaceutique d'oxaliplatine d'oxaliplatine selon l'une des revendications précédentes, **caractérisée en ce que** la solution liquide d'oxaliplatine se trouve au contact d'une couche interne de ladite enveloppe constituée en un matériau polypropylène.

4. Poche souple étanche à usage médical contenant une préparation pharmaceutique d'oxaliplatine selon l'une des revendications précédentes, **caractérisée en ce que** la concentration en oxaliplatine de la préparation pharmaceutique est comprise entre 1 et 8 mg/ml.

5. Poche souple étanche à usage médical contenant une préparation pharmaceutique d'oxaliplatine selon la revendication 4, **caractérisée en ce que** ladite concentration est comprise entre 1 et 5 mg/ml.

6. Poche souple étanche à usage médical contenant une préparation pharmaceutique d'oxaliplatine selon l'une des revendications précédentes, **caractérisée en ce que** cette poche souple est constituée de deux feuilles soudées de matériau multicouche en feuille comprenant un film de polyamide de l'adde 11-amino-un-décanoïque lié par l'une au moins de ses faces à un film de polypropylène au moyen d'un film de polyoléfine, les films de polypropylène formant la paroi interne de la poche souple étanche.

7. Poche souple étanche à usage médical contenant une préparation pharmaceutique d'oxaliplatine selon l'une des revendications précédentes, **caractérisée en ce que** cette poche souple est multi compartimentée.

8. Poche souple étanche à usage médical contenant une préparation pharmaceutique d'oxaliplatine selon la revendication 7, **caractérisée en ce que** les multi compartiments sont définis de manière à permettre le dosage d'une préparation prête à l'emploi.

9. Poche souple étanche à usage médical contenant une préparation pharmaceutique d'oxaliplatine selon l'une des revendications précédentes, **caractérisée en ce que** cette solution présente un pH de 4,5 à 6, une teneur en oxaliplatine dans la préparation d'au moins 95 % de la teneur initiale, ainsi qu'un aspect limpide incolore exempt de précipité après conservation pendant une durée pharmaceutiquement acceptable.

## Claims

1. Flexible impervious bag for medical use containing a pharmaceutical preparation of oxaliplatinum in liquid form, wherein said flexible bag is constructed from plastic material, excluding, for the said material in direct contact with the said pharmaceutical preparation, PVC-based material.

2. Flexible impervious bag for medical use containing a pharmaceutical preparation of oxaliplatinum according to Claim 1, wherein the envelope of said bag has a multi-layer structure.

3. Flexible impervious bag for medical use containing a pharmaceutical preparation of oxaliplatinum according to one of the foregoing Claims, wherein the liquid solution of oxaliplatinum is in contact with an internal layer of the said envelope consisting of a polypropylene material.

4. Flexible impervious bag for medical use containing a pharmaceutical preparation of oxaliplatinum according to one of the foregoing Claims, wherein the concentration of oxaliplatinum in the pharmaceutical preparation is between 1 and 8 mg/ml.

5. Flexible impervious bag for medical use containing a pharmaceutical preparation of oxaliplatinum according to Claim 4, wherein said concentration is between 1 and 5 mg/ml.

6. Flexible impervious bag for medical use containing a pharmaceutical preparation of oxaliplatinum according to one of the foregoing Claims, wherein said flexible bag consists of two welded sheets of multi-layer sheet material comprising one film of polyamide of 11-amino-undecanoic acid bonded by at least one of its surfaces to a film of polypropylene by means of a film of polyolefine, the polypropylene films forming the internal wall of the impervious flexible bag.

7. Flexible impervious bag for medical use containing a pharmaceutical preparation of oxaliplatinum according to one of the foregoing Claims, wherein said flexible bag is multi-compartmented.

8. Flexible impervious bag for medical use containing a pharmaceutical preparation of oxaliplatinium according to Claim 7, wherein the multicompartments are defined in such a way as to allow the dosing of a ready-for-use preparation.

9. Flexible impervious bag for medical use containing a pharmaceutical preparation of oxaliplatinum according to one of the foregoing Claims, wherein said solution has a pH of 4.5 to 6.0, a concentration of oxaliplatinum in the preparation of at least 95 % of the initial concentration, as well as a clear, colorless appearance free from precipitate after storage for a pharmaceutically acceptable period.

## Patentansprüche

1. Undurchlässige nachgiebige Tasche zum medizinischen Gebrauch, die ein pharmazeutisches Präparat aus Oxaliplatin enthält, das sich in flüssiger Form befindet, **dadurch gekennzeichnet, daß** die nachgiebige Tasche aus Kunststoff ausgeführt ist, wobei für denjenigen Stoff, das sich in direktem Kontakt mit dem pharmazeutischen Präparat befindet, einen Stoff aus der Basis von PVC ausgeschlossen ist.

2. Undurchlässige nachgiebige Tasche zum medizinischen Gebrauch, die ein pharmazeutisches Präparat aus Oxaliplatin enthält, nach Anspruch 1, **dadurch gekennzeichnet, daß** die Hülle der Tasche einen mehrschichtigen Aufbau hat.

3. Undurchlässige nachgiebige Tasche zum medizinischen Gebrauch, die ein pharmazeutisches Präparat aus Oxaliplatin enthält, nach einem der vorhergehenden Ansprüche, **dadurch gekennzeichnet, daß** die flüssige Oxaliplatin-Lösung sich in Kontakt mit einer Innenschicht der Hülle befindet, die aus Polypropylen-Material gebildet ist.

4. Undurchlässige nachgiebige Tasche zum medizinischen Gebrauch, die ein pharmazeutisches Präparat aus Oxaliplatin enthält, nach einem der vorhergehenden Ansprüche, **dadurch gekennzeichnet, daß** die Oxaliplatin-Konzentration des pharmazeutischen Präparats zwischen 1 und 8 mg/ml liegt.

5. Undurchlässige nachgiebige Tasche zum medizinischen Gebrauch, die ein pharmazeutisches Präparat aus Oxaliplatin enthält, nach Anspruch 4, **dadurch gekennzeichnet, daß** die Konzentration zwischen 1 und 5 mg/ml liegt.

6. Undurchlässige nachgiebige Tasche zum medizinischen Gebrauch, die ein pharmazeutisches Präparat aus Oxaliplatin enthält, nach einem der vorhergehenden Ansprüche, **dadurch gekennzeichnet, daß** diese nachgiebige Tasche aus zwei verschweißten Folien aus mehrschichtigem Folienmaterial gebildet ist, das einen Polyamid-Film aus 11-Amino-Undecansäure enthält, der zumindest an einer seinen Flächen mit einem Polypropylen-Film mittels eines Polyolefin-Films verbunden ist, wobei die Polypropylen-Filme die Innenwand der undurchlässigen nachgiebigen Tasche bilden.

7. Undurchlässige nachgiebige Tasche zum medizinischen Gebrauch, die ein pharmazeutisches Präparat aus Oxaliplatin enthält, nach einem der vorhergehenden Ansprüche, **dadurch gekennzeichnet, daß** diese nachgiebige Tasche vielfach abgeteilt ist.

8. Undurchlässige nachgiebige Tasche zum medizinischen Gebrauch, die ein pharmazeutisches Präparat aus Oxaliplatin enthält, nach Anspruch 7, **dadurch gekennzeichnet, daß** die Vielfach-Abteilungen derart bestimmt sind, daß die Dosierung eines gebrauchsfertigen Präparats ermöglicht wird.

9. Undurchlässige nachgiebige Tasche zum medizinischen Gebrauch, die ein pharmazeutisches Präparat aus Oxaliplatin enthält, nach einem der vorhergehenden Ansprüche, **dadurch gekennzeichnet, daß** diese Lösung einen pH-Wert von 4,5 bis 6, einen Gehalt an Oxaliplatin im Präparat von wenigstens 95 % des Anfangsgehalts sowie ein farbloses klares Aussehen frei von Niederschlag nach Konservierung während einer pharmazeutisch annehmbaren Dauer aufweist.
